Europäisches Patentamt

**European Patent Office**

**Office européen des brevets**

(11) Numéro de publication: **0 128 821**
A1

(12) **DEMANDE DE BREVET EUROPEEN**

(21) Numéro de dépôt: **84401154.4**

(22) Date de dépôt: **06.06.84**

(51) Int. Cl.³: **B 29 F 1/10,** B 29 F 1/022, A 43 B 13/37, A 43 B 9/16

(30) Priorité: **08.06.83 FR 8309498**

(43) Date de publication de la demande: **19.12.84**
**Bulletin 84/51**

(84) Etats contractants désignés: **AT BE CH DE FR GB IT LI LU NL SE**

(71) Demandeur: **Etablissements DELHOMMEAU ET COMPAGNIE S.A., Avenue de Nantes, F-44116 Vieillevigne (FR)**
Demandeur: **S.A.R.L. MANUFACTURE FRANCAISE DES CHAUSSURES ERAM, F-49110 Saint Pierre Montlimart (FR)**
Demandeur: **Société Anonyme BROSSARD S.A., Route de Thouars, F-79700 Mauleon (FR)**

(72) Inventeur: **Brossard, Philippe, Lot de la Borderie, F-87300 Bellac (FR)**
Inventeur: **Biotteau, Gérard, Les Zinnias, F-49110 Saint Pierre Monlimart (FR)**
Inventeur: **Brossard, Jacques, 8 Cité de Loge, F-79700 Mauleon (FR)**

(74) Mandataire: **Lemonnier, André, Cabinet LEMONNIER 4, Boulevard Saint-Denis, F-75010 Paris (FR)**

(54) Procédé de moulage d'objets en matière thermoplastique ou thermodurcissable comportant un insert, son application à la fabrication par injection de bloc semelle-talon pour chaussures et moule pour la mise en oeuvre du procédé.

(57) La présente invention concerne un procédé de moulage d'objets en matière thermoplastique ou thermodurcissable comportant un insert, notamment au moulage d'une semelle de chaussure comportant un talon avec un insert périphérique prémoulé, cet insert étant solidarisé avec la semelle par la matière thermoplastique injectée constituant la semelle et un moule pour la mise en oeuvre du procédé.

Le procédé consiste à utiliser l'insert (7) pour contrôler la diffusion du flux de la matière injectée dans la cavité (8) du moule, de manière à retarder sa venue en contact avec les parois de la cavité du moule qui sont à une température accélérant la transformation liquide-solide de la matière injectée. Dans le cas de la fabrication d'une semelle, le canal d'injection débouche dans un canal (21) traversant le talon et le flux est dévié par le cambrion (20).

1

**Procédé de moulage d'objets en matière thermoplastique ou thermodurcissable comportant un insert,son application à la fabrication par injection de blocs semelle-talon pour chaussures et moule pour la mise en oeuvre du procédé.**

La présente invention concerne d'une manière générale le moulage d'objets en matière thermoplastique, le procédé s'étendant également aux matières plastiques thermodurcissables.

On sait que,dans le procédé de moulage d'une matière thermoplastique, la matière chaude et fluide est injectée dans un moule dans lequel elle se refroidit à un degré suffisant pour qu'elle soit solidifiée au moins sur la majorité de son volume et pour que l'objet moulé puisse être retiré du moule sans présenter des défauts de surface dus au retrait de la partie de la masse insuffisamment gélifiée. Pour pouvoir retirer l'objet du moule aussi rapidement que possible afin d'accroître la cadence d'utilisation du moule, on refroidit le moule mais un moule froid provoque une gélification accélérée qui s'oppose au remplissage des parties du moule situées les plus loin de la buse d'injection et l'on sait, par exemple, que les premières pièces injectées à la reprise du travail alors que le moule n'a pas encore atteint sa température optimale présentent de tels défauts. Il faut donc, de manière connue, placer la buse d'injection aussi près que possible du centre de gravité de la pièce à mouler et la température du moule est un compromis entre une vitesse de refroidissement de la matière suffisamment faible pour obtenir un bon remplissage et une vitesse de refroidissement élevée assurant une cadence de moulage élevée.

Un autre facteur important dans le processus de moulage est la section de passage offerte à la matière chaude entre le début et la fin de l'injection. En effet, autour de la buse la matière se trouve gélifiée sur une épaisseur qui croît avec le temps et donc réduit la section libre pour le fluage de la matière chaude injectée destinée à remplir les parties les plus éloignées de la cavité du moule. Ceci entraîne diverses conditions d'épaisseur minima de la pièce et on recherche, selon l'art connu, une vitesse d'injection élevée sous une pression élevée pour que toutes les parties soient remplies presque simultanément et une épaisseur constante pour que toutes les parties de la pièce présentent au moment de l'enlèvement de la pièce la même structure en épaisseur, savoir les mêmes gradients du degré de gélification en tous les points de la surface.

Ces conditions de moulage obligent, pour abaisser la durée de remplissage, à réduire le poids de la pièce moulée.

Les mêmes problèmes se posent dans l'injection dans un moule chaud d'une résine thermodurcissable.

On connaît, d'autre part, de nombreux objets en matière plastique moulée qui comportent des inserts, l'insert qui peut être noyé dans la masse de l'objet et/ou qui peut constituer une partie de la surface de l'objet moulé, étant placé dans le moule avant fermeture. Des exemples de tels inserts sont particulièrement nombreux dans l'industrie de la chaussure dans laquelle les blocs semelle-talon destinés à être soudés sous la tige ou les semelles réalisées par injection directe sous la tige,sont obtenus par injection de matière thermoplastique. De tels inserts peuvent notamment constituer un enrobage de la surface extérieure du talon, des éléments noyés constituant le cambrion ou autres.

La présente invention résulte de l'observation que,dans ces fabrications,un nouvel insert est mis en place dans le moule avant chaque injection d'un objet et se trouve donc à une température qui peut être notablement différente de celle du moule. En outre, ces inserts sont souvent prémoulés en matière plastique rigide et présentent un coefficient d'isolation thermique assez élevé.

La présente invention a pour but de maîtriser la gélification ou prise de la matière thermoplastique ou thermodurcissable injectée dans la cavité du moule de manière à éviter une gélification précoce qui s'opposerait au remplissage.

Ce but est atteint,conformément à l'invention,par le fait que l'on utilise l'insert pour contrôler la diffusion du flux de la matière injectée dans la cavité du moule, de manière à retarder sa venue en contact avec les parois de la cavité du moule qui sont à une température accélérant la transformation liquide-solide de la matière injectée.

Cette diffusion peut être contrôlée en ménageant dans l'insert une conduite ou un réseau de conduites qui prolongent la buse d'injection pour aboutir en divers points de l'insert répartis dans le volume du moule.

Une application typique de ce premier mode de réalisation de l'invention est le cas d'une semelle de chaussure comportant un talon avec un insert périphérique en polystyrène ou résine ABS imitant le cuir, cet insert étant solidarisé avec la semelle par la matière thermoplastique injectée constituant la semelle. Si la matière plastique est injectée comme habituellement à travers le pavé dans le cas d'un bloc semelle prémoulé ou à travers la coquille dans le cas d'une semelle moulée par injection directe sous la tige, des problèmes de remplissage correct de la cavité prévue dans l'insert pour sa solidarisation avec la masse de matière thermoplastique de la semelle peuvent se poser parce que la matière commence à se gélifier au contact des parois froides entourant la buse d'injection et on constate souvent des manques de matière.

Dans une telle fabrication et conformément au procédé de l'invention, on utilise un moule présentant une cavité à la forme du bloc semelle-talon et met en place dans la partie correspondant au talon un insert s'adaptant étroitement à la cavité du moule, ce talon comportant un canal traversant reliant la surface supérieure de l'insert à sa base, et on injecte la totalité de la masse de matière remplissant la cavité de moulage correspondant à la semelle et au canal du talon par la base de la partie de la cavité du moule correspondant au talon, en utilisant le canal traversant comme canal d'injection pour remplir la partie de la cavité correspondant à la semelle. Avec ce mode de réalisation la matière injectée ne commence à se refroidir rapidement qu'au sortir du canal traversant de l'insert lorsqu'elle vient au contact de la partie métallique du moule.

La diffusion peut également être contrôlée en munissant l'insert de surfaces déflectrices qui dévient les courants de matière injectée vers les parties les moins réactives, respectivement les parties les moins froides ou les moins chaudes du moule sèlon qu'il s'agit d'une matière thermoplastique ou thermodurcissable. Cette caractéristique peut être combinée avec la première notamment dans le cas où une partie du moule n'est pas refroidie, par exemple dans le cas de la fermeture du moule par une forme portant la tige et la première de montage d'une chaussure dont on moule la semelle. Dans un tel cas on munit l'insert de talon d'un cambrion qui dévie une partie du flux provenant de la conduite dudit insert vers la surface de la première de montage garnissant la face de la forme fermant le moule.

Enfin l'insert peut comporter dans sa surface faisant face à une surface refroidie ou chauffée du moule un canal ou un réseau de canaux en creux qui réservent un cheminement à la matière fluide lorsque les couches au contact du moule se trouvant dans la même section sont déjà gélifiées.

L'invention permet, avec un débit de matière injectée donné, d'injecter un plus grand volume sans risque de défauts de remplissage ou de réduire ou d'élever la température du moule afin d'accélérer la gélification ou la prise et d'augmenter la cadence.

Le moule pour la mise en oeuvre du procédé conforme à l'invention est caractérisé en ce qu'il comporte des moyens pour loger et fixer dans la cavité du moule un insert comportant au moins un canal traversant de façon que ce canal soit raccordé au canal d'injection du moule.

Le moule pour la fabrication d'un bloc semelle-talon pour chaussure présente, de façon connue, une cavité à la forme du bloc semelle-talon à obtenir, une partie de cette cavité s'adaptant à la forme de l'insert du talon et il est caractérisé en ce que la partie de la cavité correspondant à la semelle se prolonge au-dessus de la partie de la cavité correspondant au talon, le canal d'injection débouchant au fond de la cavité correspondant au talon.

Selon une autre caractéristique le canal d'injection se prolonge à l'intérieur de la cavité du talon par une cheminée d'injection.

Selon un mode réalisation préférentiel et pour permettre de fixer des talons de formes différentes sous la même semelle, donc d'utiliser des inserts de formes différentes, une pièce interchangeable est montée dans la partie de la cavité du moule correspondant au talon pour adapter la cavité du moule à la forme de l'insert.

Dans un tel cas et pour assurer un raccordement correct du canal de l'insert avec la cheminée terminant le canal d'injection, la cheminée d'injection est de préférence portée par un bloc amovible avec des canaux assurant la liaison entre ladite cheminée et le canal d'injection.

Du fait de l'adaptation serrée de l'insert dans la cavité de moulage, l'opération de démoulage pourrait s'avérer difficile, aussi et selon une autre caractéristique de l'invention, le moule est muni d'un canal d'injection d'air comprimé débouchant au voisinage du fond de la cavité du talon.

On décrira ci-après un exemple de mise en oeuvre du procédé de l'invention pour la fabrication de chaussures par injection directe avec référence aux dessins ci-annexés dans lesquels:

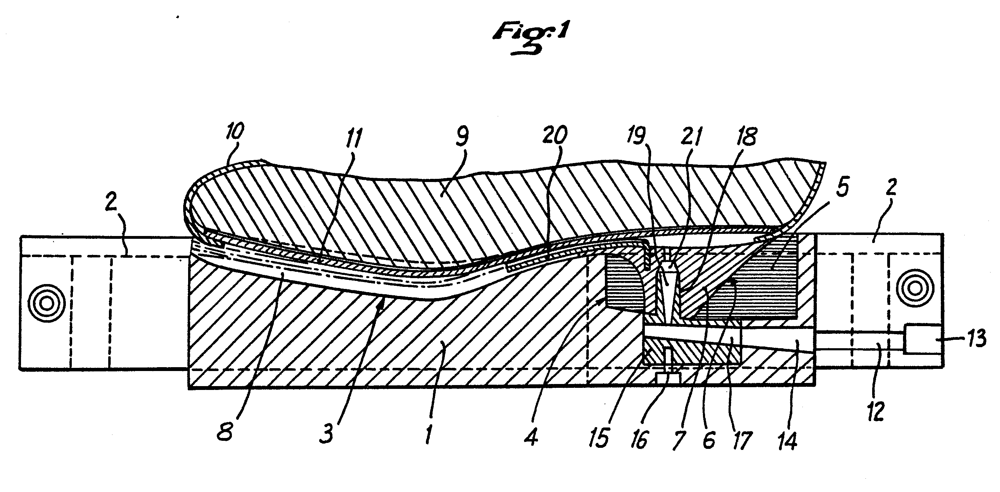

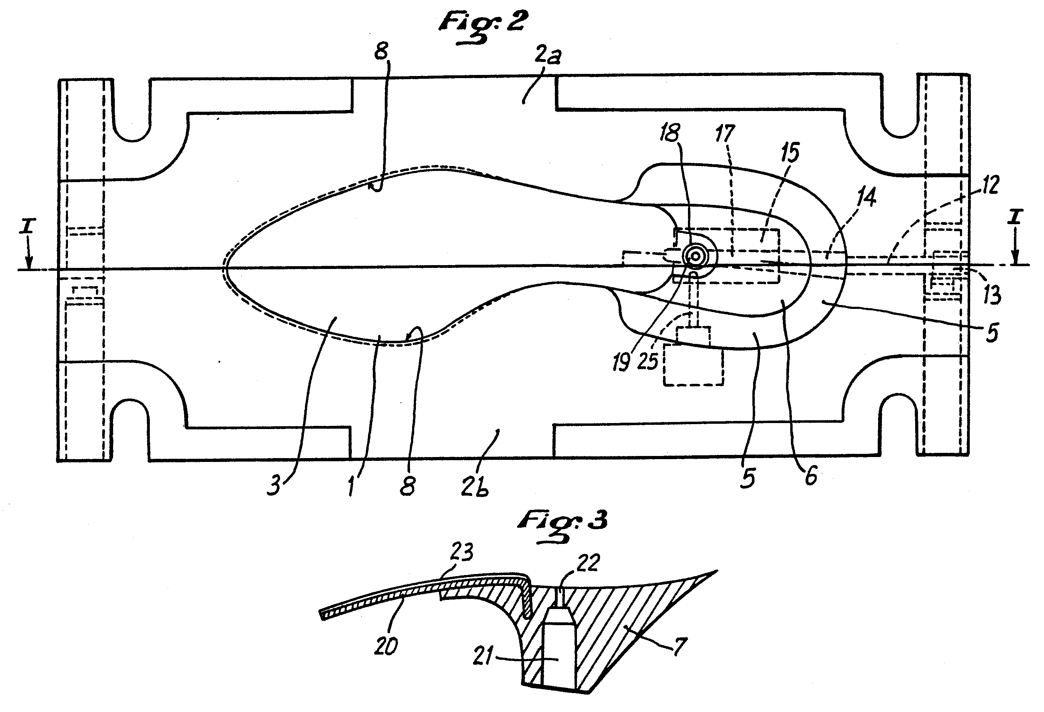

La figure 1 est une vue en coupe axiale du moule fermé avant injection par I-I de figure 2; la figure 2 est une vue en plan du moule proprement dit et la figure 3 est une vue en coupe de détail de l'insert constitué par le talon et le cambrion.

Dans ces dessins la référence 1 désigne le pavé du moule qui s'adapte étroitement dans une couronne constituée par deux demi-coquilles 2a-2b. La surface supérieure du pavé comporte une surface 3 délimitant la face inférieure du patin de la semelle et, en arrière de celle-ci, une cavité 4 dans laquelle est logée et maintenue par des moyens non représentés une fourrure interchangeable 5 qui présente elle-même une cavité 6 dont la surface avant se raccorde à la surface 3 et dont la forme correspond étroitement à la forme de l'insert de talon 7 qui, d'une façon connue, forme au moins la surface extérieure visible du talon. Les demi-coquilles 2 délimitent par leurs parois internes 8 les faces latérales de la semelle.

La cavité du moule est fermée par une forme 9 sur laquelle est montée une tige 10 et sa première de montage 11. Le bord de la tige est serré entre la forme 9 et les arêtes supérieures des parois internes 8 des demi-coquilles 2.

Conformément à l'invention, l'injection dans la cavité du moule est effectuée par un canal d'injection 12 avec un embout de raccordement 13, le canal 12 et l'embout 13 étant formés par des cavités hémi-cylindriques dans les faces en contact des deux demi-coquilles 2. Le canal 12 se prolonge par un canal 14 dans le pavé 1 et par des canaux dans un organe d'injection interchangeable 15. Ce bloc d'injection 15 est fixé dans une cavité de forme correspondante au fond de la cavité 4 du pavé, par exemple par une vis 16. Il présente un canal 17 prolongeant le canal 14 et une cheminée 18 qui vient en saillie dans la cavité 4 en étant parallèle à la direction de démoulage. Cette cheminée 18 comporte un canal tronconique 19 qui se raccorde par sa petite base avec le canal 17 pour assurer un cisaillement automatique de la carotte d'injection.

L'interchangeabilité du bloc d'injection 15 et de la fourrure 5 permet d'utiliser le même moule pour des chaussures ayant la même forme de semelle mais des talons, donc des inserts, 7 de formes différentes.

Conformément à l'invention, l'insert 7 qui est constitué dans le présent cas par un talon prémoulé, par exemple en poly (acrylonitrile-butadiène-styrène), assemblé avec un cambrion 20, comporte un canal traversant 21 conformé pour venir s'emboîter exactement sur la cheminée d'injection 18. Ce canal 21 débouche par un orifice de faible section 22 sur la face supérieure de l'insert 2 dans la cavité de moulage. Le cambrion 20 est placé en avant de ce débouché et a une section transversale qui forme une gouttière 23 dirigée vers la surface inférieure de la forme.

Dans un tel moule, le pavé 1 et les coquilles 2 sont en général refroidis soit par des circulations d'eau internes, soit par contact direct avec des platines elles-mêmes refroidies. Les surfaces 3 et 11 sont donc relativement froides alors que les surfaces constituées par la première de montage 11, l'insert de talon 7 et le cambrion 20 sont à la température ambiante et réalisées en des matériaux moins bons conducteurs de la chaleur que le moule métallique.

La matière thermoplastique chaude, en général du chlorure de polyvinyle, injectée par le canal 21 est, à sa sortie, dirigée vers la surface de la première de montage 11 et le flux est également dévié vers celle-ci par la gouttière 23. La gélification est plus rapide au contact des surfaces 3 et 11 et progresse à partir de celles-ci vers l'intérieur de la cavité de moulage de sorte que l'on peut obtenir un bon remplissage de la cavité du moule, même si les parois 3 et 11 sont surrefroidies parce que la gouttière 23 du cambrion 20 forme un canal d'injection qui est relativement isolé thermiquement et réchauffé par échange thermique avec le flux de matière chaude, canal qui aboutit sensiblement au centre de gravité de la cavité d'injection.

Pour faciliter le démoulage qui pourrait être rendu difficile du fait que l'insert 7 est engagé à force dans la fourrure 5 et peut se trouver bloqué par sa dilatation thermique, un canal 25 (Fig. 2) débouche au fond de la cavité 6 et il peut être relié à une source d'air comprimé pour éjecter le talon 7 de la cavité 6.

La chaussure est terminée par fixation d'un bonbout, ce bonbout étant fixé par emboîtement à force de sa tige cylindrique creuse dans la cavité laissée au démoulage par la cheminée d'injection 18.

Revendications

1. Procédé de moulage d'objets en matière thermoplastique
ou thermodurcissable comportant un insert,
caractérisé en ce que l'on utilise l'insert pour contrôler
la diffusion du flux de la matière injectée dans la cavité
du moule, de manière à retarder sa venue en contact avec
les parois de la cavité du moule qui sont à une température
accélérant la transformation liquide-solide de la matière
injectée.

2. Un procédé selon la revendication 1,
caractérisé en ce que la diffusion du flux de la matière
injectée dans la cavité du moule est contrôlée en ménageant
dans l'insert une conduite ou un réseau de conduites qui
prolongent la buse d'injection pour aboutir en divers points
de l'insert répartis dans le volume du moule.

3. Un procédé selon la revendication 1,
caractérisé en ce que la diffusion du flux de la matière
injectée dans la cavité du moule est contrôlée en munissant
l'insert de surfaces déflectrices qui dévient les courants
de matière injectée vers les parties les moins réactives,
respectivement les parties les moins froides ou les moins
chaudes du moule selon qu'il s'agit d'une matière thermoplastique ou thermodurcissable.

4. Application du procédé selon l'une quelconque des revendications 1 à 3 au moulage d'une semelle de chaussure comportant
un talon avec un insert périphérique prémoulé, cet insert
étant solidarisé avec la semelle par la matière thermoplastique
injectée constituant la semelle,
caractérisée en ce qu'on utilise un moule présentant une
cavité (8) à la forme du bloc semelle-talon et met en place
dans la partie correspondant au talon un insert (7) s'adaptant
étroitement à la cavité du moule, ce talon comportant un

çanal traversant (21-22) reliant la surface supérieure de l'insert à sa base, et on injecte la totalité de la masse de matière remplissant la cavité de moulage correspondant à la semelle et au canal du talon par la base de la partie de la cavité (6) du moule correspondant au talon, en utilisant le canal traversant (21-22) comme canal d'injection pour remplir la partie (8) de la cavité correspondant à la semelle.

5. Application du procédé selon la revendication 4, au cas du moulage d'une semelle par injection directe sous une tige (10) portée par une forme (9) fermant la cavité du moule, caractérisée en ce qu'on munit l'insert (7) de talon d'un cambrion (20) qui dévie une partie du flux provenant de la conduite dudit insert vers la surface de la première de montage (11) garnissant la face de la forme (9) fermant le moule.

6. Application du procédé selon la revendication 5, caractérisée en ce que l'insert (20) comporte dans sa surface faisant face à une surface refroidie ou chauffée du moule un canal (23) ou un réseau de canaux en creux qui réservent un cheminement à la matière fluide lorsque les couches au contact du moule se trouvant dans la même section sont déjà gélifiées.

7. Un moule pour la mise en oeuvre du procédé selon la revendication 1, caractérisé en ce qu'il comporte des moyens (6) pour loger et fixer dans la cavité du moule un insert (7) comportant au moins un canal traversant (21-22) de façon que ce canal soit raccordé au canal d'injection (12-14) du moule.

8. Un moule selon la revendication 7 pour la mise en oeuvre du procédé selon les revendications 4 à 6 présentant, de façon connue, une cavité (6) à la forme du bloc semelle-talon à obtenir, une partie de cette cavité s'adaptant à la forme de l'insert (7) du talon, caractérisé en ce que la partie (8) de la cavité correspondant à la semelle se prolonge au-dessus de la partie de la cavité (4) correspondant au talon, le canal d'injection (14) débouchant au fond de la cavité correspondant au talon.

9. Un moule selon la revendication 8, caractérisé en ce que le canal d'injection (7) se prolonge à l'intérieur de la cavité (6) du talon par une cheminée d'injection (18).

10. Un moule selon la revendication 8, caractérisé en ce que, pour permettre de fixer des talons de formes différentes sous la même semelle, donc d'utiliser des inserts de formes différentes, une pièce interchangeable (5) est montée dans la partie (4) de la cavité du moule correspondant au talon pour adapter la cavité du moule à la forme de l'insert (7).

11. Un moule selon la revendication 10, caractérisé en ce que la cheminée d'injection (18) est portée par un bloc amovible (15) avec des canaux (17) assurant la liaison entre ladite cheminée (18) et le canal d'injection (14).

12. Un moule selon la revendication 8, caractérisé en ce qu'il est muni d'un canal (25) d'injection d'air comprimé débouchant au voisinage du fond de la cavité (6) du talon.

*Fig.1*

0128821

*Fig.2*

*Fig.3*

# 0128821

**Office européen
des brevets**

## RAPPORT DE RECHERCHE EUROPEENNE

EP 84 40 1154

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | EP-A-0 023 356 (ESANA SCHUHFABRIK SAILER GmbH) * page 2, lignes 27-35; page 3, lignes 1-16; page 5, lignes 8-37; page 6, lignes 1-17; figures 3-5,8,9 * | 1-3 | B 29 F 1/10<br>B 29 F 1/022<br>A 43 B 13/37<br>A 43 B 9/16 |
| Y | | 4-8 | |
| | --- | | |
| Y | FR-A-2 012 913 (M. GIANNINI) * pages 1-2; page 3, lignes 1-28; figures 4-5 * | 4-8 | |
| A | | 11 | |
| | --- | | |
| A | US-A-2 447 512 (J.F. LEAHY) * colonne 2, lignes 29-60; colonne 3, lignes 1-14; colonne 4, lignes 33-41; colonne 4, lignes 67-75; colonne 5, lignes 1-17; colonne 6, lignes 20-40; colonne 8, lignes 25-56; figures 2,3,7 * | 9 | DOMAINES TECHNIQUES RECHERCHES (Int. Cl. 3)<br><br>A 43 B<br>A 43 D<br>B 29 F |
| | --- | | |
| A | GB-A-1 189 674 (ANCO PLASTICS) * page 1, lignes 85-88; page 2, lignes 1-48; page 5, lignes 64-73; page 5, lignes 100-130; page 6, lignes 1-9; page 6, lignes 44-115; figures 3-6,8-9,11-12,16-18 * | 10 | |
| | ---     -/- | | |

Le présent rapport de recherche a été établi pour toutes les revendications

| Lieu de la recherche | Date d'achèvement de la recherche | Examinateur |
|---|---|---|
| LA HAYE | 12-09-1984 | GOURIER P.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503 03.82

Office européen
des brevets

**RAPPORT DE RECHERCHE EUROPEENNE**

0128821
Numéro de la demande

EP 84 40 1154

| DOCUMENTS CONSIDERES COMME PERTINENTS | | | Page 2 | |
|---|---|---|---|---|
| Catégorie | Citation du document avec indication, en cas de besoin, des parties pertinentes | Revendication concernée | CLASSEMENT DE LA DEMANDE (Int. Cl. ³) | |
| A | FR-A-2 248 927 (BATA INDUSTRIES LTD.) * en entier * | 9,11-12 | | |
| A | FR-A-2 105 498 (SOCIETE INTERNATIONALE DE CONSTRUCTIONS MECANIQUES) * page 1, lignes 1-15; page 2, lignes 10-40; page 3, lignes 1-10; figures 1,2 * | 12 | | |
| | --- | | DOMAINES TECHNIQUES RECHERCHES (Int. Cl. ³) | |

Le présent rapport de recherche a été établi pour toutes les revendications

| Lieu de la recherche | Date d'achèvement de la recherche | Examinateur |
|---|---|---|
| LA HAYE | 12-09-1984 | GOURIER P.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82